# EUROPEAN PATENT SPECIFICATION

(11) **EP 1 450 876 B1**
(45) Date of publication and mention of the grant of the patent: **17.08.2016**
(21) Application number: 02788469.1
(22) Date of filing: 07.11.2002
(51) Int. Cl.: A61N 1/30, A61N 1/32, A61N 1/04

(54) **INTEGRATED TRANSDERMAL DRUG DELIVERY SYSTEM**
INTEGRIERTES TRANSDERMALES ARZNEIMITTELABGABESYSTEM
SYSTEME INTEGRE D'ADMINISTRATION TRANSDERMIQUE DE MEDICAMENT

(30) Priority: 07.11.2001 US 337002 P
(43) Date of publication of application: 01.09.2004
(60) Divisional application: 11000062.7
(73) Proprietor: Syneron Medical Ltd., 20692 Yoqneam Illit (IL)
(72) Inventor: SOHN, Zeev, 44853 Ginot Shomron (IL)
(74) Representative: O'Neill, Brian
(86) International application number: PCT/IL2002/000896
(87) International publication number: WO 2003/039620

(56) References cited:
- WO-A-97/48440
- US-A- 3 163 166
- US-A- 3 163 166
- US-A- 5 603 693
- US-A- 5 908 401
- US-A- 5 908 401
- US-A- 5 919 156

## Description

### CROSS-REFERENCE TO RELATED APPLICATIONS

This application is a continuation-in-part of US published Patent Application 2002058936, entitled, "Monopolar and bipolar current application for transdermal drug delivery and analyte extraction," which is assigned to the assignee of the present patent application and incorporated herein by reference.

This application claims priority from US Provisional Patent Application 60/337,002, filed November 7, 2001, entitled, "Integrated transdermal drug delivery system," which is assigned to the assignee of the present patent application and incorporated herein by reference.

### FIELD OF THE INVENTION

The present invention relates generally to methods and devices for drug delivery and specifically to methods and devices for puncturing the outer layer of living skin and to methods and devices for transdermal drug delivery.

### BACKGROUND OF THE INVENTION

A number of different methods have been developed to perform transdermal drug delivery including passive diffusion of a drug between a skin patch and skin, as well as active processes such as iontophoresis, sonophoresis, electroporation, and chemically enhanced diffusion. These methods are primarily used for generating transdermal movement of small molecules, but generally do not enhance the motion of large molecules through the 10-50 micron thick outermost layer of the skin, the stratum corneum epidermidis.

PCT Publication WO 97/07734 to Eppstein et al., which is incorporated herein by reference, describes thermal ablation of the stratum corneum using an electrically resistive element in contact with the stratum corneum, such that a high current through the element causes a general heating of tissue in its vicinity, most particularly the stratum corneum.

Electroporation is also known in the art as a method to increase pore size by application of an electric field. This process is described in an article by Chizmadzhev et al., entitled "Electrical properties of skin at moderate voltages," Biophysics Journal, February, 1998, 74(2), 843-856. Electroporation is described as a means for transiently decreasing the electrical resistance of the stratum corneum and increasing the transdermal flux of small molecules by applying an electric field to increase the size of existing pores.

U.S. Patent 5,019,034 to Weaver et al., describes apparatus for applying high voltage, short duration electrical pulses on the skin to produce electroporation, and states that "...reversible electrical breakdown ... along with an enhanced tissue permeability, is the characteristic effect of electroporation."

U.S. Patents 5,885,211, 6,022,316, 6,142,939 and 6,173,202 to Eppstein et al., describe methods for forming micropores in the stratum corneum by heating tissue-bound water above the vapor point with a heat conducting element, so as to enhance transdermal transport of an analyte or active substance. Further enhancement techniques include the use of sonic energy, pressure, and chemical enhancers.

U.S. Patents 3,964,482 to Gerstel, 6,050,988 to Zuck, and 6,083,196 to Trautman et al., describe other apparatus and methods for facilitating transdermal movement of a substance.

U.S. Patent 6,148,232 to Avrahami, describes apparatus for applying electrodes at respective points on skin of a subject and applying electrical energy between two or more of the electrodes to cause resistive heating and subsequent ablation of the stratum corneum primarily in an area intermediate the respective points. Various techniques for limiting ablation to the stratum corneum are described, including spacing of the electrodes and monitoring the electrical resistance of skin between adjacent electrodes.

Electrosurgery is commonly used during surgical procedures today, particularly in endoscopic and laparoscopic surgery where direct access to the tissue being dissected is limited. Electrosurgery involves applying radio frequency electric current to electrodes which are used to sever tissue or achieve homeostasis. A publication entitled "Instruction Manual for the Force 2 Electrosurgical Generator" (Valleylab/Tyco Healthcare Group LP, Boulder, Colorado), describes the modes of operation of electrosurgical devices.

U.S. Patent 6,159,194 to Eggers et al., describes electrosurgical apparatus and methods for inducing tissue contraction, without ablation or dissociation of surrounding tissue, in order to reduce wrinkles in skin.

US. Patents 6,066,134 and 6,024,733 to Eggers et al., describe electrosurgical apparatus and methods for ablating outer layers of skin for the treatment of unwanted tissue pigmentations, melanomas, and other skin disorders.

U.S. Patent 6,090,106 to Goble et al., describes monopolar and bipolar electrosurgical instruments for ablating gross tissue, such as the prostate or endometrial tissue.

U.S. Patent 4,943,290 to Rexroth et al., describes electrosurgical apparatus in which a nonconductive fluid is transported to the region of an electrode in order to isolate the electrode and prevent undesirable damage of surrounding tissue. U.S. Patent 5,908,401 discloses the preamble of claim 1.

### SUMMARY OF THE INVENTION

It is an object of some aspects of the present invention to provide improved apparatus for transdermal delivery of a substance.

It is yet a further object of some aspects of the present invention to provide improved apparatus for reducing sensation and minimizing damage to skin underlying the stratum corneum during channel creation.

It is still a further object of some aspects of the present invention to provide improved apparatus for puncturing the skin and transdermally delivering a substance using a self-contained device.

It is an additional object of some aspects of the present invention to provide improved apparatus for transdermally delivering a substance using a standard substance-containing skin patch, such as a medicated skin patch.

It is still another object of some aspects of the present invention to provide improved apparatus for puncturing the skin and transdermally delivering a substance using sterile and/or safe medical techniques.

Disclosed a device for enhancing transdermal movement of a substance, such as a drug, comprises: (a) a handle, for supporting a removable electrode cartridge, which comprises at least one electrode, and which holds the electrode in a vicinity of the skin of a subject; and (b) a control unit, coupled to the handle, which causes electrical current to pass through the electrode, typically generating current flow or one or more sparks between the electrode and the stratum corneum epidermidis, in order to create at least one micro-channel in the stratum corneum and thereby enable or augment transdermal movement of the substance. The control unit comprises circuitry to control the magnitude, frequency, and/or duration of the electrical energy delivered to the electrode, so as to control current flow and/or spark generation, and thus micro-channel formation. It is emphasized that although some exemplary arrangements are described with respect to ablating the stratum corneum by spark generation, substantially any method known in the art for ablating stratum corneum may be used.

Typically, the device creates micro-channels, as described hereinbelow, as a means for enhancing the transdermal delivery of a substance from a skin patch placed on the skin after the device has been removed from the skin. The electrode cartridge is typically discarded after one use, and as such is designed for easy attachment to the handle and subsequent detachment from the handle. Preferably, to minimize the likelihood of contamination of the cartridge and its associated electrodes, attachment and detachment of the cartridge are performed without the user touching the cartridge. Preferably, cartridges are sealed in a sterile cartridge holder, which is opened immediately prior to use, whereupon the handle is brought in contact with a top surface of the cartridge, so as to engage a mechanism that locks the cartridge to the handle. Preferably, a simple means of unlocking and ejecting the cartridge, which does not require the user to touch the cartridge, is also provided.

For some applications, the device is adapted to mark or indicate the region of the skin where micro-channels have been created, such that a substance-containing patch, such as a medicated patch, can be precisely placed over the treated region of the skin after the electrode cartridge has been removed from the skin. Alternatively or additionally, visible dimples are temporarily formed on the skin by protrusive elements on the cartridge. For some applications, one or more of the electrodes are shaped to form the protrusive elements. Alternatively, the protrusive elements are integrated into the outer surface of the handle, or elsewhere on the device. It is noted that micro-channel generation (when practiced in accordance with the techniques described in the above-cited US Patent 6,148,232 to Avrahami or US Patent 6,708,060 to Avrahami and Sohn, entitled, "Handheld apparatus and method for transdermal drug delivery and analyte extraction," which are assigned to the assignee of the present patent application and incorporated herein by reference) does not generally leave any visible mark, because even the large number of micro-channels typically generated are not associated with large swaths of ablated skin.

Preferably, a substance-containing patch is placed over the treated region by partially pulling back a cover of the patch, which protects the patch before use. This exposes an adhesive region adjacent to one edge of the patch, and the adhesive region is placed adjacent to the marked region of skin. Once the adhesive region is fixed to the skin, continued removal of the cover places the patch across the treated region of skin. Proper adherence to usage instructions generally ensures that the patch can be placed in a sterile manner, if desired.

For other applications, the patch comprises: (a) an adhesive cut-out template which is placed on the skin, and through which the cartridge is placed to treat the region of skin exposed through the template, and (b) a substance-containing pad, such as a medicated pad, attached to the template, which is to be placed over the treated region of skin. In these applications, after removing a protective backing, the template portion of the patch is placed on the skin and secured by the adhesive. An electrode cartridge is affixed to the handle, the user holds the handle so as to place the cartridge against the region of skin inside the template, and the electrodes are energized to treat the skin. The electrode cartridge is removed from the skin, after which a protective covering is removed from the pad by pulling on a tab projecting from the covering, so as to concurrently lift and place the pad over the treated region of skin. Preferably, but not necessarily, the electrode cartridge is subsequently discarded. It is noted that the integration of the template and the patch into a single unit assists the user in accurately placing the pad onto the treated area of skin.

For still other applications, an integrated electrode/substance-containing pad cartridge is used, to provide an additional easy-to-use device. In these applications, the handle is used to pick up and secure the cartridge to the handle as in the previous applications, but the cartridge now comprises both the electrode array and the pad; no template is typically used. The user places the electrodes against the skin and activates a power switch, such as a button, on the handle to initiate electrical treatment of the skin. An adhesive strip, coupled to the bottom of the pad, comes in contact with and sticks to the skin when the electrodes are pressed against the skin. A top cover on the pad is coupled to the electrode region of the cartridge, such that as the electrode region, fixed to the handle, is removed from the skin the top cover is pulled off the pad and the pad is concurrently folded over the treated region of skin. In this application, the user does not need to touch any parts of the electrode cartridge or the pad, so the likelihood of the user contaminating the apparatus is substantially reduced.

In some exemplary arrangements, current is applied to the skin in order to ablate the stratum corneum by heating skin cells. In some exemplary arrangements, spark generation, cessation of spark generation, and/or a specific current level is used as a form of feedback, which indicates that the desired depth has been reached and current application should be terminated. For these applications, the electrodes are preferably shaped and/or supported in a cartridge that is conducive to facilitating ablation of the stratum corneum to the desired depth, but not beyond that depth. Alternatively, the current is configured so as to ablate the stratum corneum without the generation of sparks.

In general, preferred embodiments of the present invention typically incorporate apparatus described in US published Patent Application 2002058936 to Avrahami and Sohn, entitled, "Monopolar and bipolar current application for transdermal drug delivery and analyte extraction," which is assigned to the assignee of the present patent application. For example, the '645 application describes maintaining the ablating electrodes either in contact with the skin, or up to a distance of about 500 microns therefrom. The '936 published patent application further describes spark-induced ablation of the stratum corneum by applying a field having a frequency between about 10 kHz and 4000 kHz, preferably between about 10 kHz and 500 kHz.

Alternatively or additionally, preferred embodiments of the present invention incorporate apparatus described in US Patent 6,708,060 to Avrahami and Sohn, filed April 23, 2001, entitled, "Handheld apparatus and method for transdermal drug delivery and analyte extraction," which is assigned to the assignee of the present patent application. Still further alternatively or additionally, preferred embodiments of the present invention incorporate methods and apparatus described in the above-cited US Patent 6,148,232 to Avrahami.

The term "micro-channel" as used in the context of the present patent application refers to a pathway generally extending from the surface of the skin through all or a significant part of the stratum corneum, through which pathway molecules can diffuse. Preferably, micro-channels allow the diffusion therethrough of large molecules at a greater rate than the same molecules would diffuse through pores generated by electroporation.

In some preferred embodiments of the present invention, the cartridge supports an array of electrodes, preferably closely-spaced electrodes, which act together to produce a high micro-channel density in an area of the skin under the cartridge. Typically, however, the overall area of micro-channels generated in the stratum corneum is small compared to the total area covered by the electrode array.

For some applications, a user applies the device to himself, in which case the "user" and the "subject," as used herein, are the same person.

In further exemplary arrangements, a concentric electrode set is formed by employing the skin contact surface of the cartridge as a return path for the current passing from the electrode array to the skin. The cartridge has a relatively large contact surface area with the skin, resulting in relatively low current densities in the skin near the cartridge, and thus no significant heating or substantial damage to the skin at the contact surface. In proximity to each electrode in the electrode array, by contrast, the high-energy applied field typically induces very rapid heating and ablation of the stratum corneum.

There is therefore provided, in accordance with a preferred embodiment of the present invention, apparatus for facilitating delivery of a substance through skin of a subject, including:
a handle; and
a cartridge, removably coupled to the handle, the cartridge including one or more electrodes and a patch including the substance, the electrodes adapted to be applied to a region of the skin, and the patch adapted to be applied to at least a portion of the region of the skin by removal of the electrodes therefrom.

In an exemplary arrangement, the electrodes are adapted, when a current is applied therethrough, to create at least one micro-channel in stratum corneum epidermidis of the skin.

In an embodiment, the substance includes a drug, and the apparatus is adapted to facilitate delivery of the drug through the skin of the subject.

In an exemplary arrangement, at least one of the electrodes is adapted to contact the skin to create a contact area therewith having a characteristic diameter of less than about 200 microns.

In an exemplary arrangement, at least one of the electrodes includes a bipolar electrode.

In an exemplary arrangement, the cartridge is adapted to include only a single dose of the substance.

In an embodiment, the cartridge includes an electrical connector, adapted to electrically couple the cartridge to the handle.

In an embodiment, the electrodes are adapted, when a current is applied therethrough, to ablate stratum corneum of the skin.

In an embodiment, the patch includes an adhesive strip on a skin-contact surface thereof, the adhesive adapted to adhere to the skin of the subject at a time when the one or more electrodes are applied to the region of the skin.

In an embodiment, the patch includes:
a protective covering, which includes a tab coupled to the cartridge; and
a pad including the substance, the pad adapted to be brought into contact with at least a portion of the skin when the tab is pulled.

In an exemplary arrangements, the handle includes a coupling mechanism, adapted to lock the cartridge to the handle when the handle and cartridge are brought into contact with one another.

In an exemplary arrangements, the handle includes a switch, and the apparatus is adapted to allow coupling of the cartridge to the handle only when the switch is activated.

In an exemplary arrangements, the cartridge includes at least one support, and the handle is shaped to define a slot therein, adapted to receive and be coupled to the support.

In an exemplary arrangements, the cartridge includes a coupling mechanism, adapted to lock the cartridge to the handle when the handle and cartridge are brought into contact with one another. In an exemplary arrangements, the coupling mechanism includes at least one support, and the handle is shaped to define a slot therein, adapted to receive and be coupled to the support.

In an exemplary arrangement, the cartridge is adapted so that a ratio of a total contact area of the electrodes with the skin to an area of the region of the skin is less than about 1:20. In an exemplary arrangement, the cartridge is adapted so that the ratio is less than about 1:200.

In an exemplary arrangement, the handle includes a status indicator. In an embodiment, the status indicator is adapted to indicate to a user of the apparatus to remove the electrodes from the region of the skin, thereby applying the patch to the portion of the region.

In an embodiment, the apparatus includes a control unit, adapted to apply a current between two or more of the electrodes when the electrodes are in contact with the region of the skin, and adapted to configure the current to cause ablation of stratum corneum in the region, so as to facilitate passage of the substance from the patch through the ablated stratum corneum. In an exemplary arrangement, the control unit is adapted to apply the current responsive to a measurement of electrical impedance between two or more of the electrodes. In an exemplary arrangement, the handle includes a pressure-sensitive switch, adapted to be activated when the handle is pressed against the skin, and the control unit is adapted to apply the current responsive to the activation of the pressure-sensitive switch. In an exemplary arrangement, the handle includes an override switch, and the control unit is adapted to withhold applying the current when the override switch is activated, notwithstanding activation of the pressure switch.

In an exemplary arrangement, the control unit is adapted to apply an alternating current between the two or more electrodes. In an exemplary arrangement, the control unit is adapted to apply, between the two or more electrodes, an alternating current having a frequency of at least 50 kHz.

In an exemplary arrangement, the two or more electrodes include a return electrode and two or more current-driving electrodes, the control unit is adapted to apply respective currents between each of the current-driving electrodes and the return electrode, and the return electrode is adapted to provide a return path for the current, substantially without ablating stratum corneum in a vicinity of the return electrode. In an exemplary arrangement, the return electrode is at least ten times larger than the current-driving electrodes.

There is also provided, in accordance with exemplary arrangement apparatus for facilitating delivery of a substance through skin of a subject, including:
a handle;
a patch including the substance; and
a cartridge, removably coupled to the handle, the cartridge including one or more electrodes, the electrodes adapted to be applied to a region of the skin,
wherein the apparatus includes a marking unit, adapted to apply a marking to the skin indicative of a position for placement of the patch following removal of the electrodes from the region of the skin.

In an exemplary arrangement, the marking unit includes an inkpad, adapted to apply a marking substance to the skin so as to provide the marking.

In an exemplary arrangement, the marking unit includes one or more protrusive elements, adapted to press the skin so as to temporarily provide the marking.

In an exemplary arrangement, the apparatus includes a pressure-sensitive switch, adapted to be activated when the apparatus is pressed against the skin.

There is further provided, in accordance with a preferred embodiment of the present invention, apparatus for facilitating delivery of a substance through skin of a subject, for use with a handle, the apparatus including:
a cartridge, adapted to be removably coupled to the handle, the cartridge including one or more electrodes which are adapted, when a current is applied therethrough, to cause ablation of stratum corneum of a region of the skin; and
a patch coupled to the cartridge and including the substance, adapted to be applied to at least a portion of the region of the skin,
the cartridge and patch being configured such that when the electrodes are brought in contact with the region of the skin and subsequently removed, the patch is brought into a position to cover at least a portion of the region.

In an embodiment, the patch includes an adhesive strip, coupled to an edge thereof, and adapted to adhere to the skin of the subject at a time when the one or more electrodes are applied to the skin.

In an embodiment, the patch includes:
a pad including the substance; and
a protective covering, which includes a tab, the tab adapted to be coupled to the cartridge such that removal of the cartridge from the skin by the handle causes the tab to be pulled, thereby removing the protective covering from the pad and bringing the pad into contact with at least a portion of the region.

In an exemplary arrangement, the cartridge includes a coupling mechanism, adapted to lock the cartridge to the handle when the handle and cartridge are brought into contact with one another.

In an embodiment, the cartridge includes an electrical connector, adapted to electrically couple the cartridge to the handle.

There is still further provided, in accordance with exemplary arrangement, apparatus for facilitating delivery of a substance through skin of a subject, including a patch assembly having a first portion and a second portion adjacent to the first portion,
the first portion including a frame that surrounds an open portion of the patch assembly, such that a region of the skin is exposed through the open portion when the first portion is placed on the skin, and
the second portion including a pad including the substance, the pad being adapted to be brought into contact, through the open portion, with at least a portion of the region of the skin.

In an exemplary arrangement, the frame includes an adhesive on a skin-contact surface thereof, the adhesive adapted to adhere to the skin of the subject.

In an exemplary arrangement, the second portion includes a protective covering, which includes a tab, and the pad is adapted to be brought into contact with the portion of the skin when the tab is pulled.

In an exemplary arrangement, the first and second portions are adapted to articulate at respective edges coupling the portions.

There is also provided, in accordance with an exemplary arrangement, apparatus for facilitating delivery of a substance through skin of a subject, including:
a handle;
a cartridge, removably coupled to the handle, the cartridge including one or more electrodes; and
a patch including:
   the substance;
   an indicator, adapted to indicate a region of the skin to which the cartridge is to be applied by the handle, the region substantially adjacent to the patch; and
   an adhesive strip, coupled to an edge of the patch, and adapted to adhere to the skin of the subject at a time when the one or more electrodes are applied to the skin.

In an exemplary arrangement, the indicator includes at least one of:
a shaped portion of the patch, the shaped portion being indicative of the region of the skin to which the cartridge is to be applied, and
a symbol on the patch, the symbol being indicative of the region of the skin to which the cartridge is to be applied.

In an embodiment, the patch includes:
a protective covering, which includes a tab; and
a pad including the substance, the pad adapted to be brought by a user into contact with at least a portion of the region of the skin when the tab is pulled, after the user has removed the cartridge from the region.

There is provided, in accordance with an exemplary arrangement, apparatus for facilitating delivery of a substance through skin of a subject from within the body of the subject, including:
a handle; and
a cartridge, removably coupled to the handle, the cartridge including one or more electrodes and a patch, the electrodes adapted to be applied to a region of the skin, and the patch adapted to be applied to at least a portion of the region of the skin by removal of the electrodes therefrom, and to absorb the substance.

In an exemplary arrangement, the substance includes an analyte, and the patch is adapted to absorb the analyte.

There is yet additionally provided, in accordance with a preferred embodiment of the present invention, apparatus for facilitating delivery of a substance through skin of a subject, including:
a handle; and
a cartridge, removably coupled to the handle, the cartridge including one or more skin preparation elements and a patch including the substance, the skin preparation elements adapted to be applied to a region of the skin, and the patch adapted to be applied to at least a portion of the region of the skin by removal of the skin preparation elements therefrom.

There is provided, in accordance with an exemplary arrangement, apparatus for facilitating delivery of a substance through skin of a subject, including:
a handle;
a patch including the substance; and
a cartridge, removably coupled to the handle, the cartridge including one or more skin preparation elements, the skin preparation elements adapted to be applied to a region of the skin,
wherein the apparatus includes a marking unit, adapted to apply a marking to the skin indicative of a position for placement of the patch following removal of the skin preparation elements from the region of the skin.

There is further provided, in accordance with an exemplary arrangement, apparatus for facilitating delivery of a substance through skin of a subject, for use with a handle, the apparatus including:
a cartridge, adapted to be removably coupled to the handle, the cartridge including one or more skin preparation elements; and
a patch coupled to the cartridge and including the substance, adapted to be applied to at least a portion of the region of the skin,
the cartridge and patch being configured such that when the skin preparation elements are brought in contact with the region of the skin and subsequently removed, the patch is brought into a position to cover at least a portion of the region.

There is yet further provided, in accordance with an exemplary arrangement, apparatus for facilitating delivery of a substance through skin of a subject from within the body of the subject, including:
a handle; and
a cartridge, removably coupled to the handle, the cartridge including one or more skin preparation elements and a patch, the skin preparation elements adapted to be applied to a region of the skin, and the patch adapted to be applied to at least a portion of the region of the skin by removal of the skin preparation elements therefrom, and to absorb the substance.

In an exemplary arrangement, the one or more skin preparation elements include micro-needles, adapted to puncture stratum corneum of the skin.

In an exemplary arrangement, the one or more skin preparation elements include electrodes, adapted to perform electroporation on the skin.

In an exemplary arrangement, the one or more skin preparation elements include a sonophoresis unit, adapted to perform sonophoresis on the skin.

In an exemplary arrangement, the one or more skin preparation elements are adapted to ablate stratum corneum of the skin.

In an exemplary arrangement, the one or more skin preparation elements are adapted to apply laser energy to the stratum corneum, so as to ablate the stratum corneum.

There is still further provided, in accordance with an exemplary arrangement, a method for facilitating delivery of a substance contained in a patch through skin of a subject, including:
removably coupling, to a handle, a cartridge including a plurality of electrodes;
placing at least a portion of the cartridge over the skin so that at least a portion of the electrodes are applied to a region of the skin;
driving a current between two or more of the electrodes; and
applying the patch to at least a portion of the region of the skin by removing the electrodes from the region of the skin.

There is yet further provided, in accordance with an exemplary arrangement, a method for facilitating delivery of a substance contained in a patch through skin of a subject, including:
removably coupling, to a handle, a cartridge including a plurality of electrodes;
placing at least a portion of the cartridge over the skin so that at least a portion of the electrodes are applied to a region of the skin;
driving a current between two or more of the electrodes;
marking the skin, with at least one of: the handle and the cartridge, to indicate a position on the skin for applying the patch;
removing the electrodes from the region of the skin; and
applying the patch to at least a portion of the region of the skin, responsive to the marking of the skin.

There is also provided, in accordance with an exemplary arrangement, a method for facilitating delivery of a substance contained in a patch through skin of a subject, including:
removably coupling, to a handle, a cartridge including a plurality of electrodes;
placing the patch on a first region of the skin, so that an indicator located on the patch indicates a location of a second region of the skin, the second region substantially adjacent to the first region;
adhering the patch to the first region;
responsive to the indicator, placing at least a portion of the cartridge over the second region of the skin so that at least a portion of the electrodes are applied to the second region;
driving a current between two or more of the electrodes; and
applying the patch to at least a portion of the second region.

There is additionally provided, in accordance with an exemplary arrangement, a method for facilitating delivery of a substance through skin of a subject from within the body of the subject, including:
removably coupling, to a handle, a cartridge including a plurality of electrodes;
placing at least a portion of the cartridge over the skin so that at least a portion of the electrodes are applied to a region of the skin;
driving a current between two or more of the electrodes;
applying the patch to at least a portion of the region of the skin by removing the electrodes from the region of the skin; and
absorbing the substance into the patch.

There is yet additionally provided, in accordance with an exemplary arrangement, a method for facilitating delivery of a substance contained in a patch through skin of a subject, including:
removably coupling, to a handle, a cartridge including a plurality of skin preparation elements;
placing at least a portion of the cartridge over the skin so that at least a portion of the skin preparation elements are applied to a region of the skin;
activating the skin preparation elements so as to prepare the skin for the delivery of the substance; and
applying the patch to at least a portion of the region of the skin by removing the skin preparation elements from the region of the skin.

There is still additionally provided, in accordance with an exemplary arrangement, a method for facilitating delivery of a substance contained in a patch through skin of a subject, including:
removably coupling, to a handle, a cartridge including a plurality of skin preparation elements;
placing at least a portion of the cartridge over the skin so that at least a portion of the skin preparation elements are applied to a region of the skin;
activating the skin preparation elements so as to prepare the skin for the delivery of the substance;
marking the skin, with at least one of: the handle and the cartridge, to indicate a position on the skin for applying the patch;
removing the skin preparation elements from the region of the skin; and
applying the patch to at least a portion of the region of the skin, responsive to the marking of the skin.

There is further provided, in accordance with an exemplary arrangement, a method for facilitating delivery of a substance through skin of a subject from within the body of the subject, including:
removably coupling, to a handle, a cartridge including a plurality of skin preparation elements;
placing at least a portion of the cartridge over the skin so that at least a portion of the skin preparation elements are applied to a region of the skin;
activating the skin preparation elements so as to prepare the skin for the delivery of the substance;
applying the patch to at least a portion of the region of the skin by removing the skin preparation elements from the region of the skin; and
absorbing the substance into the patch.

The present invention will be more fully understood from the following detailed description of the preferred embodiments thereof, taken together with the drawings, in which:

### BRIEF DESCRIPTION OF THE DRAWINGS

Fig. 1A is a schematic, sectional illustration of a device for transdermal delivery of a substance, in accordance with a preferred embodiment of the present invention;
Fig. 1B is a schematic, sectional illustration of an electrode cartridge and the attachment thereof to the device of Fig. 1A, in accordance with a preferred embodiment of the present invention;
Figs. 2A, 2B, 2C and 2D are schematic illustrations of the operation of the device of Fig. 1A for transdermal delivery of a substance, in accordance with a preferred embodiment of the present invention;
Fig. 3A is a schematic illustration of a patch for use in conjunction with the device of Fig. 3B, in accordance with a preferred embodiment of the present invention;
Fig. 3B is a schematic illustration of the operation of a device for transdermal delivery of a substance using the patch of Fig. 3A, in accordance with a preferred embodiment of the present invention;
Fig. 3C is a schematic illustration of the application of the patch of Fig. 3A, in accordance with a preferred embodiment of the present invention;
Fig. 4 is a schematic illustration of a patch for use with some or all of the devices shown herein, in accordance with a preferred embodiment of the present invention;
Fig. 5 is a schematic illustration of a combined electrode array/patch for use with a transdermal substance-delivery device, in accordance with a preferred embodiment of the present invention; and
Figs. 6A, 6B and 6C are schematic illustrations of the operation of yet another device for transdermal delivery of a substance, in accordance with a preferred embodiment of the present invention.

### DETAILED DESCRIPTION OF PREFERRED EMBODIMENTS

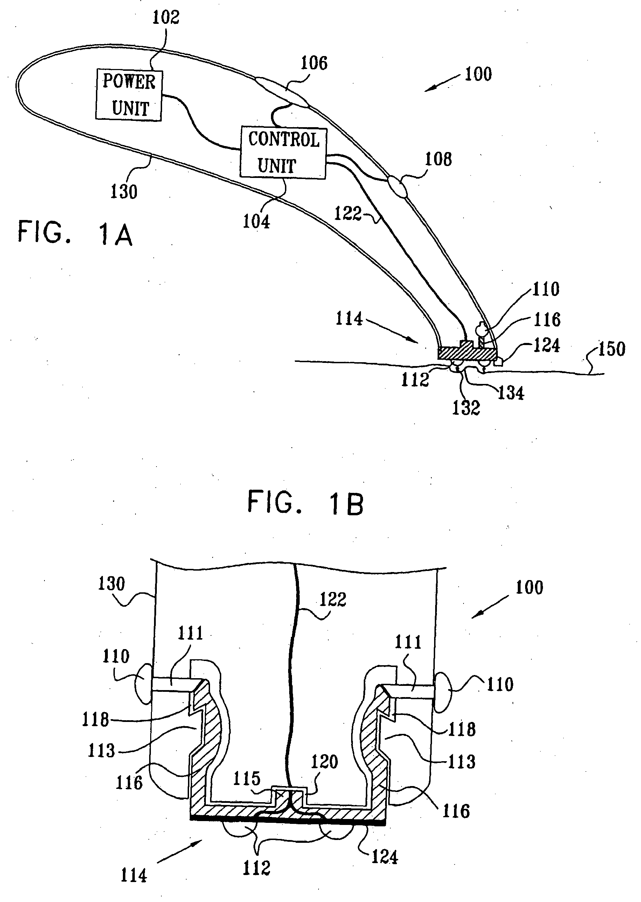

Fig. 1A is a schematic, sectional illustration of a device 100 for facilitating transdermal delivery of a substance, such as a drug, in accordance with a preferred embodiment of the present invention. Device 100 comprises a handle 130, a power unit 102, and a control unit 104. Additionally, handle 130 is mechanically coupled to an electrode cartridge 114, comprising one or more electrodes 112. Preferably, electrode cartridge 114 is discarded after a single use, while device 100 is used for multiple procedures. Although handle 130 is shown in Fig. 1A and other figures as an elongated element, this is for the sake of illustration only; embodiments of the present invention include other shapes. Furthermore, the length of handle 130 can be greater than the width of the handle (as shown in the figures), equal to the width of the handle, or less than the width of the handle. In addition, although handle 130, power unit 102, and control unit 104 are shown in the figures as incorporated in an integrated unit, this is for the sake of illustration only. In some exemplary arrangements, power unit 102 and/or control unit 104 are located external to handle 130 and are coupled to handle 130 over wires or wirelessly.

Preferably, control unit 104 is electrically coupled to electrode cartridge 114 by an electrical bus 122, so as to drive a current through electrodes 112 typically configured to cause current flow or one or more sparks 132 to occur between electrodes 112 and skin 150 of a subject. Activation of device 100 is initiated when a user activates a power switch 106, such as a button, which is preferably located at a convenient position on handle 130. Handle 130 comprises a status indicator 108, such as a status light or an audio tone generator, which informs the user of the status of device 100, for example when the device is ready to commence electrical treatment and/or when the electrical treatment has been completed.

In an exemplary arrangement, regulation of the magnitude and timing of the voltage applied to electrodes 112 controls the strength and number of sparks 132, which are used to ablate stratum corneum so as to improve transdermal delivery of a substance, such as a drug. In an exemplary arrangement, an alternating voltage is applied to electrodes 112, such that a series of sparks 132 occur during a given time interval, with each successive spark ablating more of the stratum corneum. The alternating voltage is applied to electrodes 112 such that ablating current is driven into the stratum corneum, substantially without the generation of sparks. The alternating voltage has a frequency between about 10 kHz and 4000 kHz, preferably between about 10 kHz and 500 kHz.

In an exemplary arrangement, a high-magnitude DC voltage is applied to electrodes 112. Preferably, the degree of ablation is controlled by the strength of the applied voltage, the number of sparks 132 generated, and/or the length of time the voltage is applied.

Each one of electrodes 112 has a contact area with the skin characterized by a diameter of approximately 10-100 microns, typically approximately 60-100 microns. It is noted that this contact area is significantly smaller than that typically used for electroporation applications. For some applications, electrodes 112 function as monopolar electrodes, whereby electrical energy is discharged from electrodes 112 into skin 150, while the return path of the electrical current passes through a much larger surface area (e.g., a metal base surrounding or adjacent to electrodes 112). This typically results in a set of distinct and non-overlapping ablated regions 134 near each electrode 112, but substantially no damage to tissue in other regions between the various electrodes. For example, each ablated area may have a characteristic diameter of about 60-100 microns, and be separated by a distance of about 700-1000 microns from adjacent ablated areas. In an exemplary arrangement for an electrode array which covers a total skin area A, the percentage of skin area A which is ablated is less than about 5%, and is typically on the order of about 0.5%.

Alternatively or additionally, when a larger number of electrodes 112 are included in device 100 (e.g., three or more), it may be desirable for one of electrodes 112 to act as an ablating electrode at a particular time, and for two or more of the other electrodes to act, in combination, as non-ablating "return" electrodes, each conveying a fraction of the ablating current back to control unit 104. Further alternatively or additionally, two relatively large return electrodes are provided, to obtain the safety and other benefits known in the art to be associated with "split grounds." Still further alternatively or additionally, one or more pairs of electrodes 112 are driven in a bipolar mode, in which or current is driven or spark 132 occurs between electrodes 112 in a pair, resulting in ablation of skin 150 in a region thereof.

It is to be appreciated that although some exemplary arrangements are described herein with respect to generating sparks in order to ablate the stratum corneum, the scope of the present invention includes driving a current through the stratum corneum in order to cause the ablation thereof by RF or other means for heating. For some applications, the methods and apparatus of the present disclosure are carried out in combination with other techniques known in the art for ablating the stratum corneum, such as those described in the above-cited PCT Publication WO 97/07734 to Eppstein et al.

Preferably, electrode cartridge 114 or handle 130 comprises a marking unit 124, which marks the treated region of skin 150 such that a substance-containing patch can be accurately and directly placed on the electrically-treated region after the cartridge has been removed from the region. In an exemplary arrangement, marking unit 124 comprises an inkpad, adapted to apply a marking substance, such as ink, to the skin in order to mark the treated region of the skin. Alternatively or additionally, marking unit 124 comprises one or more protrusive elements, which, when pressed against the skin, leave temporary dimples on the skin surrounding the treated region of skin 150, thereby facilitating the accurate placement of a substance-containing patch. The protrusive elements can be of essentially any shape that is configured to leave a temporary dimple essentially without puncturing the skin (e.g., a point, a line, a circle, etc.). For some applications, one or more of electrodes 112 are shaped to form the protrusive elements.

Fig. 1B is a schematic, sectional illustration of the coupling of electrode cartridge 114 to handle 130 of device 100. Cartridge 114 is designed to be easy to attach to handle 130 by simply: (a) lining up cartridge supports 116 with slots 118 in handle 130, and (b) pressing on handle 130 so as to force supports 116 into slots 118. Preferably, supports 116 each comprise an indentation into which a protuberance 113 in each of slots 118 fits, so as to securely lock electrode cartridge 114 to handle 130. Additionally, the mating of supports 116 and slots 118 leads to an electrical connection between control unit 104 and electrodes 112 via electrical bus 122. Preferably, a male connector 115 on electrode cartridge 114 couples to a female connector 120 on handle 130, ensuring a proper connection. Preferably, the dimensions of the surface of cartridge 114 that comprises electrodes 112 are about 2 cm to about 5 cm on a side, and the length of supports 116 is about 2 mm to about 10 mm.

Electrode cartridge 114 is preferably ejected from handle 130 by pressing two cartridge eject buttons 110, which displace two corresponding rods 111 and cause supports 116 to bend inward. This releases the lock created by protuberance 113 fitting into the indentation in each support 116. In a preferred embodiment, the force transmitted to supports 116 by rods 111 acts to eject cartridge 114 from handle 130, and it is therefore not necessary for the user to touch the electrode cartridge after it has been used.

As appropriate, some or all of the following features may be incorporated into device 100 or the other devices described hereinbelow, so as to simplify or optimize performance of the device:

A button may be included on the handle, which is pressed by the user in order to allow the cartridge to be loaded onto the handle. The control unit may be programmed to disallow current flow to the electrodes when this button is being pressed. Alternatively, the handle may be configured such that pressing this button mechanically blocks current from flowing through the electrodes, e.g., by creating an open circuit.

A button may be included on the handle, which is pressed by the user in order to allow the cartridge to be loaded onto the handle. For some applications the control unit is programmed to initiate the ablating process responsive to a pressure-sensitive switch on the handle indicating that the handle is being pressed against the skin (e.g., as described in the above-cited US published Patent Application 2002058936 to Avrahami and Sohn). Preferably, the control unit is programmed to ignore signals from the pressure-sensitive switch while the button is being pressed, in order to eliminate false indications that the electrodes are being pressed against the skin. Alternatively, the handle may be configured such that pressing the button prevents the pressure-sensitive switch from generating a reading indicative of the electrodes being pressed against the skin. For these applications, power switch 106 is preferably (but not necessarily) eliminated, and the output of the pressure-sensitive switch is used to signal the control unit to initiate ablation.

In combination with the pressure-sensitive switch and/or the power button, or separately therefrom, a measurement of the electrical impedance between some of the electrodes may be made in order to determine whether the impedance measurements are indicative of the handle being in position on the skin of the subject.

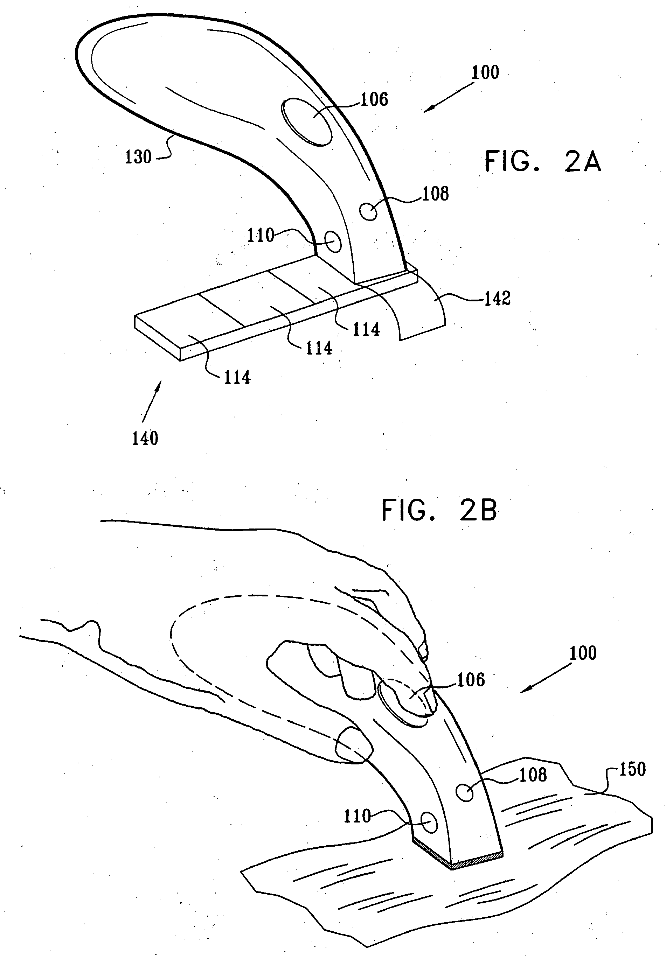

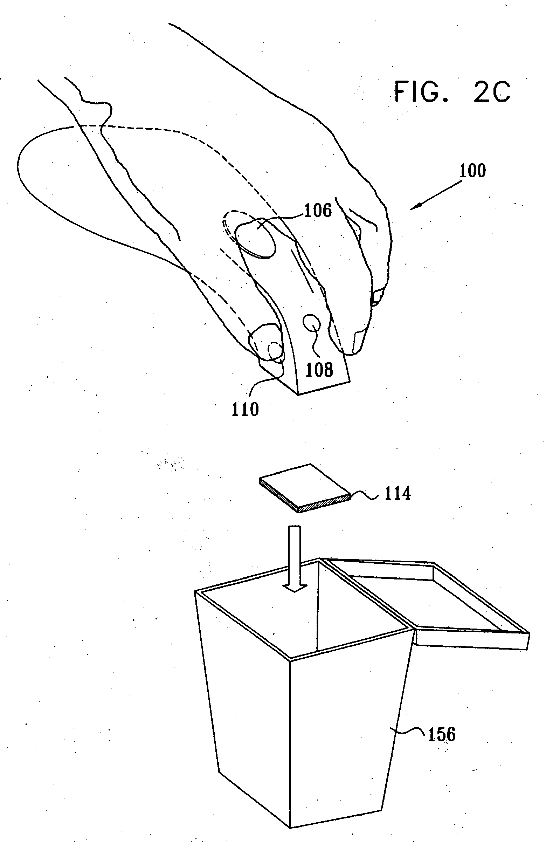

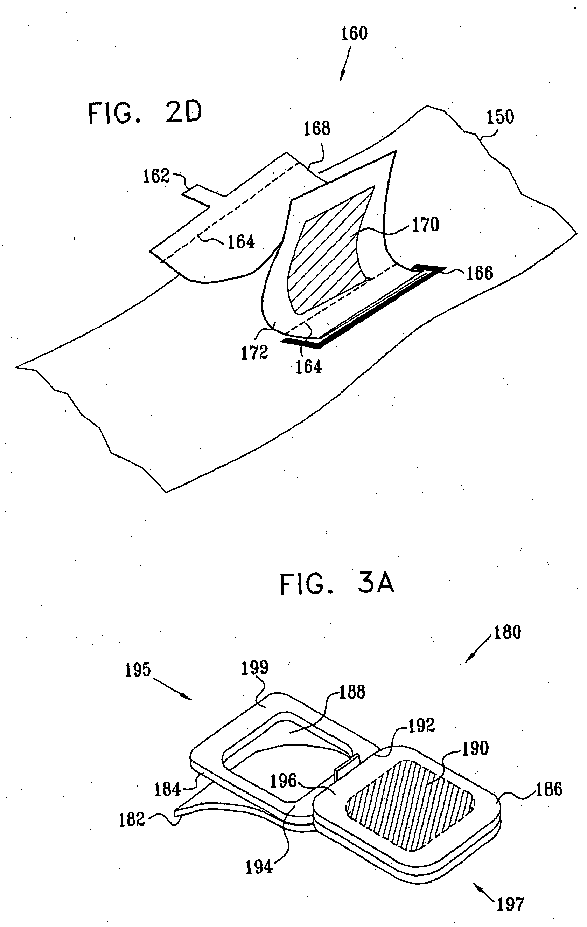

Figs. 2A, 2B, 2C and 2D are schematic illustrations of means for using device 100 for facilitating transdermal delivery of a substance, such as a drug, in accordance with a preferred embodiment of the present invention. In an optimized treatment program, electrode cartridge 114 is discarded after a single use, while device 100 is used multiple times. Thus, a plurality of cartridges 114 is preferably packaged in a cartridge holder 140, as shown in Fig. 2A. Each electrode cartridge 114 is packaged in holder 140 in a sterile manner and sealed to prevent contamination after packaging is complete.

Preferably, each cartridge 114 is covered by its own protective cover 142. To access an electrode cartridge 114, the user first removes cover 142 from the cartridge. The cartridge is arranged in holder 140 such that electrodes 112 are facing down while cartridge supports 116 (Fig. 1B) are facing up, and thus visible to the user. Grasping handle 130 of device 100, the user aligns supports 116 with slots 118 and presses down on cartridge 114 to lock the cartridge to the handle, as described above in reference to Fig. 1B. The user then removes the electrode cartridge from holder 140. It is noted that the user does not need to touch cartridge 114, particularly the underside thereof, so the cartridge remains in its sterile condition.

Electrical treatment of a region of skin 150 is accomplished by placing cartridge 114 on the skin and activating power switch 106, as illustrated in Fig. 2B. Preferably, status indicator 108 informs the user when the cartridge is properly arranged on the skin and it is appropriate to press the power button. (For example, control unit 104 may measure the electrical impedance between the various electrodes 112, and actuate status indicator 108 when the readings are indicative of firm contact between the electrodes and the skin.) Alternatively or additionally, status indicator 108 signals when the electrical treatment is complete and cartridge 114 may be removed from contact with the skin. Cartridge 114 is then removed from handle 130 by depressing buttons 110, as described above with reference to Fig. 1B, and preferably discarded in a waste receptacle 156, as illustrated in Fig. 2C.

Fig. 2D shows the placement of a patch 160, such as a medicated patch, over the treated region of skin 150 subsequent to electrical treatment as described hereinabove. Proper placement of patch 160 is preferably facilitated by the presence on skin 150 of a demarcation line 166, such as an ink line placed by marking unit 124 when the unit comprises an inkpad, or a line of dimples left by marking unit 124 when the unit comprises protrusive elements (Figs. 1A and 1B) during electrical treatment of the skin. Patch 160 comprises a substance-containing portion 170, such as a medicated portion, surrounded by an adhesive portion 172, so that the substance-containing portion can be held in contact with the electrically-treated region of skin 150. Patch 160 also comprises a removable backing 168 to maintain the sterility and efficacy of the patch prior to use. Preferably, the thickness of patch 160 is between about 0.05 mm and about 5 mm.

Preferably, patch 160 is placed on skin 150 by first creasing the patch along a fold line 164, and then separating backing 168 from the patch up to the fold line with the aid of a tab 162. The exposed adhesive portion 172 of patch 160 is aligned with ink line 166 and pressed to skin 150. Continued pulling on tab 162 simultaneously removes backing 168 from the patch and pulls the substance-containing portion over the treated region of skin 150. If necessary, slight pressure is applied to patch 160 to securely affix the patch to the skin. It is important to note that sterility has been maintained during the entire treatment process.

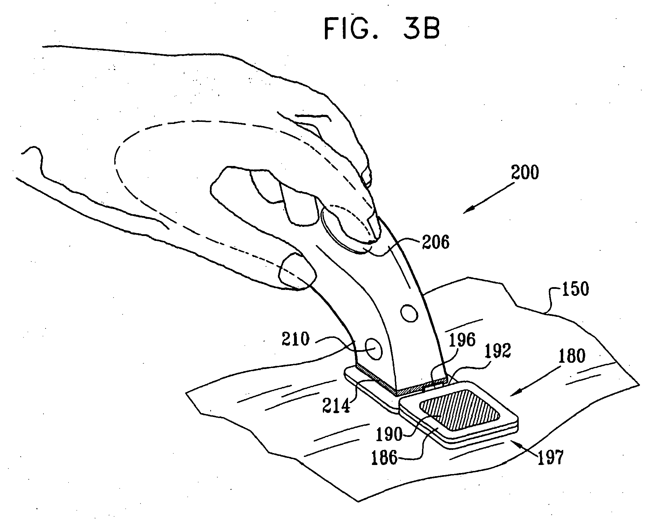

Fig. 3A is a schematic illustration of a substance-containing patch 180, such as a medicated patch, preferably to be used in conjunction with a device 200 (shown in Fig. 3B) for facilitating transdermal delivery of a substance, such as a drug, in accordance with a preferred embodiment of the present invention. Patch 180 comprises a lower backing 182, a pad 194, and an upper protective cover 186 covering pad 194. Preferably, pad 194 has two sections: (a) an electrical treatment side 195, having a shaped portion such as a frame 199 that surrounds an open portion 188 passing therethrough, and (b) a substance-treatment side 197, having a substance-containing region 190, such as a medicated region. Lower backing 182 covers an adhesive region 184 surrounding open portion 188 on the underside of electrical treatment side 195 (which underside comes in contact with skin 150). Upper cover 186 protects the upper portion of pad 194, including substance-containing region 190, from contamination. Upper cover 186 comprises a tab 196, connected to an edge 192 near open portion 188. Preferably, the thickness of patch 180 is between about 0.05 mm and about 5 mm.

Fig. 3B illustrates use of patch 180 in conjunction with device 200 for facilitating transdermal delivery of a substance, in accordance with a preferred embodiment of the present invention. Device 200 functions in essentially the same manner as device 100 described hereinabove with reference to Figs. 1A and 1B. Device 200 comprises a control unit (not shown), which drives electrodes on an electrode cartridge 214 so as to ablate skin 150, such that transdermal delivery of a substance is facilitated.

Proper placement of substance-containing region 190 of patch 180 is assisted by the design of the patch. After separating lower backing 182 from patch 180, the patch is placed on a region of skin 150. For some applications, the region is cleaned and dried prior to application of the patch. Electrode cartridge 214 is placed through open portion 188 onto the region of skin 150 exposed through the open portion. Device 200 is then used to ablate the exposed region of skin by activating a power switch 206. As discussed hereinabove with reference to electrode cartridge 114 of Figs. 2A, 2B and 2C, electrode cartridge 214 is stored in a sterile condition and is attached to device 200 in a manner that maintains the sterile condition of the cartridge. Cartridge 214 is ejected from device 200 by pressing eject buttons 210, and is typically discarded after one application.

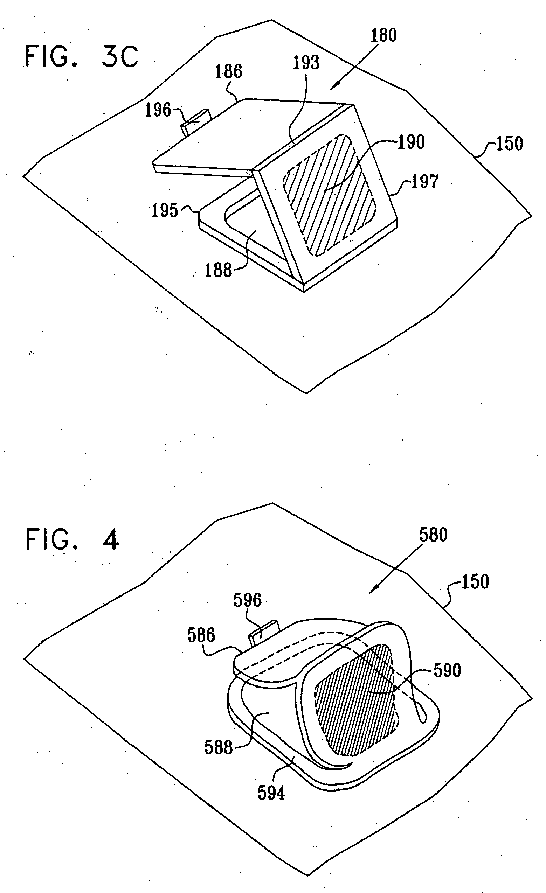

Fig. 3C is a schematic illustration of the application of patch 180, in accordance with a preferred embodiment of the present invention. After electrode cartridge 214 has been removed from open portion 188, tab 196 is pulled up and generally in the direction of open portion 188. This pulling action removes upper protective cover 186 from substance-containing region 190. Simultaneously, because upper protective cover 186 is connected to substance-treatment side 197 at edge 193 of side 197, this pulling action causes substance-treatment side 197 to fold up and over electrical-treatment side 195, bringing substance-containing region 190 into contact with skin 150 through open portion 188. As a result, the substance is applied to substantially the same region of the skin to which the electrical treatment was applied. It is noted that the entire procedure is typically performed without the user touching the electrode cartridge or the substance-containing portion of the patch.

Fig. 4 is a schematic illustration of a patch 580 for use with some or all of the devices described herein, in accordance with a preferred embodiment of the present invention. Except as specifically noted or shown in the figure, patch 580 is generally similar to patch 180 described hereinabove, and is used in a generally similar manner. As shown in Fig. 4, once the ablating device is removed subsequent to ablation of skin 150, the user pulls on tab 596, thereby simultaneously removing a cover 586 protecting a substance-containing region 590, and pulling the substance-containing region over the ablated region of skin 150 inside an open portion 588 defined by a frame 594, in a manner similar to that described above with reference to Fig. 3C. It is noted that the entire procedure is typically performed without the user touching the electrode cartridge or the substance-containing portion of the patch.

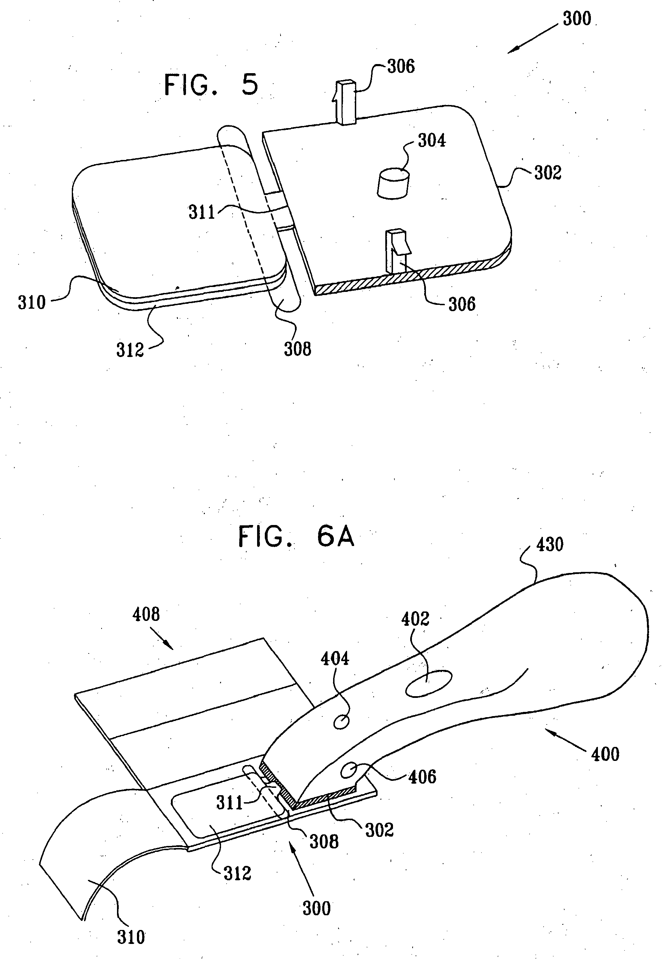

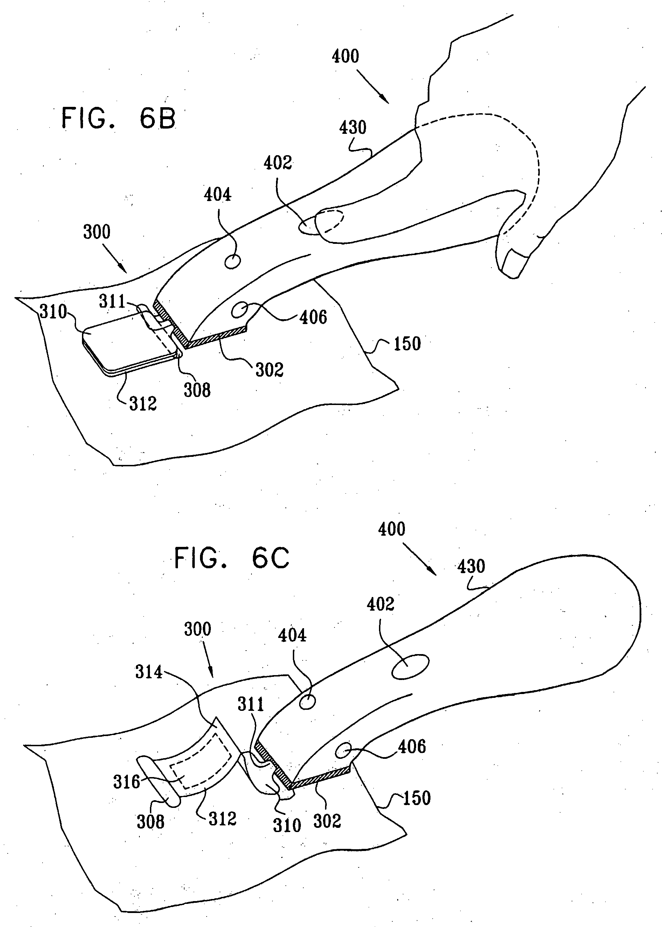

Reference is now made to Figs. 5, 6A, 6B, and 6C. Fig. 5 is a schematic illustration of a combined electrode array/patch cartridge 300 to be used in conjunction with yet another device 400 for facilitating transdermal delivery of a substance, such as a drug, in accordance with a preferred embodiment of the present invention. Figs. 6A, 6B and 6C are schematic illustrations of the use of device 400 to facilitate transdermal delivery of a substance, in accordance with a preferred embodiment of the present invention. Preferably, the thickness of cartridge 300 is between about 1 mm and about 10 mm.

Cartridge 300 comprises an electrode-containing portion 302 and a substance-containing patch 312, such as a medicated patch, integrated into a single unit (Fig. 5). Electrode-containing portion 302 comprises two cartridge supports 306, which guide and secure attachment of portion 302 to a handle 430 of device 400. In addition, portion 302 comprises a cartridge electrical connector 304 for electrically coupling electrodes (not shown) on the underside of the cartridge to handle 430. Preferably, patch 312 is covered by a protective liner 310, and the protective liner is coupled to electrode-containing portion 302 of the cartridge by a tab 311. An adhesive strip 308 is coupled to patch 312 so as to secure the cartridge to skin 150 during use. Device 400 itself preferably operates in a manner similar to that described hereinabove with reference to device 100 of Fig. 1A.

Preferably, one or more cartridges 300 are packaged in a sterile cartridge holder 408 (Fig. 6A). Peeling back a protective cover 310 allows access to an individual cartridge 300, and exposes cartridge supports 306 and electrical connector 304. Handle 430 is then aligned over cartridge 300 and pressed down upon the cartridge so as to secure the cartridge to the device, in a similar manner to that described hereinabove with reference to device 100 in Figs. 1A and 1B. Typically, no part of cartridge 300 is touched by the user during this procedure, so the sterile condition of the cartridge is generally maintained.

Holding handle 430, the user presses cartridge 300 against a region of skin 150 (Fig. 6B), where the cartridge is held in place with the aid of adhesive strip 308. Preferably, an indicator 404, such as an indicator light, coupled to handle 430 signals to the user when electrode-containing portion 302 is properly placed and ready for electrical treatment to commence. The user then activates a power switch 402, such as a power button, to initiate electrical treatment of skin 150, and the indicator preferably signals completion of the electrical treatment.

Moving device 400 away from the treated region of the skin (Fig. 6C) pulls on tab 311 of protective cover 310, tending to separate cover 310 from patch 312. Simultaneously, patch 312 is folded over the treated region of skin 150, because one side of patch 312 is held to the skin by adhesive strip 308. Eventually, cover 310 completely separates from patch 312, and a substance-containing portion 316, such as a medicated portion, of patch 312 is held in place on the treated region of skin 150 by an adhesive border 314 on patch 312. If appropriate, slight pressure is applied to patch 312 by the user to securely seat the patch on the skin. This pressure may be applied using a finger without contaminating the treated region of skin, as only the backside of the patch is touched. Electrode-containing portion 302 and cover 310 are ejected from handle 430 by pressing eject button 406 and subsequently discarded.

It is noted that the figures depicting preferred embodiments of the present invention are not necessarily drawn to scale, and, instead, change certain dimensions in order to more clearly demonstrate some aspects of the invention.

It is further noted that whereas preferred embodiments of the present invention are described herein with respect to ablating the stratum corneum to facilitate substance delivery, the scope of the present invention includes replacing the substance-containing pads with absorbing pads, so as to facilitate analyte extraction. Thus, for example, an integrated electrode array/absorbing pad may be provided in a cartridge to provide the user with an easy technique for ablating the stratum corneum and placing the absorbing pad on the ablated region to take up analyte which passes through the ablated region.

It is still further noted that whereas preferred embodiments of the present invention are described herein with respect to ablating the stratum corneum to prepare the skin for substance delivery or extraction, the scope of the present invention includes the use of other techniques for preparing the skin. Such other techniques include, but are not limited to, other electrical techniques, such as electroporation (regardless of whether such techniques ablate the skin), sonophoresis, the application of micro-needles to the skin in order to puncture the skin, and laser ablation of the skin. Embodiments of the present invention utilizing such other techniques comprise appropriate skin preparation elements, which are adapted to be applied to the skin and to prepare the skin for substance delivery or extraction, generally by creating small channels in the stratum corneum through which the substance can pass.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. Apparatus (400) for facilitating delivery of a substance through skin of a subject, comprising:
a handle (430) comprising an eject button (406); and
a cartridge (300), removably coupled to the handle (430), the cartridge (300) comprising one or more electrodes (112) in an electrode-containing portion (302) and a patch (312) comprising the substance, the electrodes (112) adapted to be applied to a region of the skin; and **characterized in that** the apparatus (400) further comprises:
a control unit (104), adapted to apply a current between two or more of the electrodes (112) when the electrodes (112) are in contact with the region of the skin, and adapted to configure the current to cause ablation of stratum corneum in the region, so as to facilitate passage of the substance from the patch (312) through the ablated stratum corneum;
*the patch (312) further comprising:*
*a substance-containing portion or pad (316), and a protective cover (310); the protective cover comprising a tab (311) adapted to be coupled to the cartridge (300); and*
*an adhesive strip (308) coupled to an edge of the patch, and adapted to adhere to the skin of the subject at a time when the one or more electrodes are applied to the skin; and*
*an adhesive border (314);*
and wherein the patch (312) is adapted such that moving the handle (430) away from the treated region of the skin pulls on *the* tab (311) of *the* protective cover (310) separating the cover (310) from the patch (312), while simultaneously causing the patch (312) to be folded over the treated region of the skin, because one side of the patch (312) is held to the skin by *the* adhesive strip (308), so that the cover (310) eventually completely separates from the patch (312) and *the* substance-containing portion (316) is held in place on the treated region of the skin (150) by *the* adhesive border (314) on the patch (312); and wherein slight pressure applied to the patch (312) by the user causes the patch on the skin to be securely seated and when the pressure is applied using a finger there is no contamination of the treated region of skin, as only the backside of the patch is touched; and wherein the electrode-containing portion (302) and cover (310) are ejected from the handle (430) by activation of *the* eject button (406) and are subsequently discarded.

2. Apparatus according to claim 1, wherein the substance includes a drug, and wherein the apparatus is adapted to facilitate delivery of the drug through the skin of the subject.

3. Apparatus according to claim 1 or 2, wherein the cartridge (300) comprises an electrical connector (304), adapted to electrically couple the cartridge (300) to the handle (430).

## Patentansprüche

1. Vorrichtung (400) zum Fördern der Zufuhr eines Stoffs durch Haut einer Versuchsperson, umfassend:
einen Griff (430), der einen Ausstoßknopf (406) umfasst; und
eine Patrone (300), die abnehmbar an den Griff (430) gekoppelt ist, wobei die Patrone (300) eine oder mehrere Elektroden (112) in einem Elektroden enthaltenden Abschnitt (302) und einen den Stoff umfassenden Patch (312) umfasst, wobei die Elektroden (112) dazu angepasst sind, an eine Region der Haut angelegt zu werden; und **dadurch gekennzeichnet, dass** die Vorrichtung (400) weiter Folgendes umfasst:
eine Steuereinheit (104), die dazu angepasst ist, einen Strom zwischen zwei oder mehr der Elektroden (112) anzulegen, wenn sich die Elektroden (112) mit der Region der Haut in Kontakt befinden, und dazu angepasst ist, den Strom dazu zu konfigurieren, Ablation von Stratum corneum in der Region zu bewirken, um den Durchgang des Stoffs von dem Patch (312) durch das abladierte Stratum corneum zu fördern;
wobei der Patch (312) weiter Folgendes umfasst:
einen Stoff enthaltenden Abschnitt oder Pad (316) und eine Schutzabdeckung (310); wobei die Schutzabdeckung eine Lasche (311) umfasst, die dazu angepasst ist, an die Patrone (300) gekoppelt zu werden; und
einen Klebestreifen (308), der an einen Rand des Patchs gekoppelt ist und dazu angepasst ist, zu einer Zeit, wenn die eine oder mehreren Elektroden an die Haut angelegt werden, an der Haut der Versuchsperson zu haften; und
eine klebende Umrandung (314);
und wobei der Patch (312) derart angepasst ist, dass das Bewegen des Griffs (430) weg von der behandelten Region der Haut an der Lasche (311) der Schutzabdeckung (310) zieht und die Abdeckung (310) von dem Patch (312) trennt, und gleichzeitig bewirkt, dass der Patch (312) über die behandelte Region der Haut gefaltet wird, da eine Seite des Patchs (312) von dem Klebestreifen (308) an der Haut gehalten wird, sodass sich die Abdeckung (310) schließlich komplett von dem Patch (312) trennt und der Stoff enthaltende Abschnitt (316) von der klebenden Umrandung (314) an dem Patch (312) ortsfest auf der behandelten Region der Haut (150) gehalten wird; und wobei von dem Anwender auf den Patch (312) ausgeübter geringfügiger Druck bewirkt, dass der Patch fest an der Haut sitzt, und wenn der Druck unter Verwendung eines Fingers ausgeübt wird, keine Verunreinigung der behandelten Region der Haut vorliegt, da nur die Rückseite des Patchs berührt wird; und wobei der Elektroden enthaltende Abschnitt (302) und die Abdeckung (310) durch Betätigung des Ausstoßknopfs (406) aus dem Griff (430) ausgestoßen werden und anschließend verworfen werden.

2. Vorrichtung nach Anspruch 1, wobei der Stoff ein Medikament umfasst, und wobei die Vorrichtung dazu angepasst ist, die Zufuhr des Medikaments durch die Haut der Versuchsperson zu fördern.

3. Vorrichtung nach Anspruch 1 oder 2, wobei die Patrone (300) einen elektrischen Verbinder (304) umfasst, der dazu angepasst ist, die Patrone (300) elektrisch an den Griff (430) zu koppeln.

## Revendications

1. Appareil (400) destiné à faciliter l'administration d'une substance à travers la peau d'un sujet, comprenant :
une poignée (430) comprenant un bouton d'éjection (406) ; et
une cartouche (300), couplée avec faculté de détachement à la poignée (430), la cartouche (300) comprenant une ou plusieurs électrodes (112) dans une partie (302) contenant des électrodes et un timbre (312) comprenant la substance, les électrodes (112) étant adaptées pour être appliquées sur une région de la peau ; et **caractérisé en ce que** l'appareil (400) comprend en outre :
une unité de commande (104), adaptée pour appliquer un courant entre deux ou plusieurs des électrodes (112) quand les électrodes (112) sont en contact avec la région de la peau, et adaptée pour configurer le courant de manière à entraîner l'ablation d'une couche cornée dans la région, afin de faciliter le passage de la substance du timbre (312) à travers la couche cornée ablatée ;
le timbre (312) comprenant en outre :
une partie ou un tampon (316) contenant une substance, et un revêtement de protection (310) ; le revêtement de protection comprenant une languette (311) adaptée pour être couplée à la cartouche (300) ; et
une bande adhésive (308) couplée à un bord du timbre, et adaptée pour adhérer à la peau du sujet lorsque les une ou plusieurs électrodes sont appliquées sur la peau ; et
une bordure adhésive (314) ;
et dans lequel le timbre (312) est adapté de telle sorte que l'écartement de la poignée (430) de la région traitée de la peau tire sur la languette (311) du revêtement de protection (310) séparant le revêtement (310) du timbre (312), et entraînant simultanément le repli du timbre (312) sur la région traitée de la peau, car un côté du timbre (312) est maintenu sur la peau par la bande adhésive (308), de telle sorte que le revêtement (310) finisse par se séparer complètement du timbre (312) et la partie (316) contenant la substance soit maintenue en place sur la région traitée de la peau (150) par la bordure adhésive (314) sur le timbre (312) ; et dans lequel une légère pression appliquée sur le timbre (312) par l'utilisateur fait en sorte que le timbre repose fermement sur la peau et quand la pression est appliquée au moyen d'un doigt la région traitée de la peau ne soit pas contaminée, puisque seulement le dos du timbre est touché ; et dans lequel la partie (302) contenant les électrodes et le revêtement (310) sont éjectés de la poignée (430) par l'activation du bouton d'éjection (406) et sont ultérieurement jetés.

2. Appareil selon la revendication 1, dans lequel la substance comporte un médicament, et dans lequel l'appareil est adapté pour faciliter l'administration du médicament à travers la peau du sujet.

3. Appareil selon la revendication 1 ou 2, dans lequel la cartouche (300) comprend un connecteur électrique (304), adapté pour coupler électriquement la cartouche (300) à la poignée (430).
